# EUROPÄISCHE PATENTSCHRIFT

(11) **EP 3 810 376 B1**
(45) Veröffentlichungstag und Bekanntmachung des Hinweises auf die Patenterteilung: **18.12.2024**
(21) Anmeldenummer: 20739936.1
(22) Anmeldetag: 09.07.2020
(51) Int. Cl.: B25J 9/00, A61H 1/02, A61H 3/00

(54) **BEWEGUNGSABHÄNGIGES STABILISIERUNGSUNTERSTÜTZUNGSSYSTEM**
MOVEMENT-DEPENDENT STABILISATION SUPPORT SYSTEM
SYSTÈME D'AIDE À LA STABILISATION EN FONCTION DES MOUVEMENTS

(30) Priorität: 10.07.2019 DE 102019210232
(43) Veröffentlichungstag der Anmeldung: 28.04.2021
(73) Patentinhaber: Kuhn, Elvira, 54294 Trier (DE); Kusmartsev, Feodor, Loughborough LE11 3JR (GB)
(72) Erfinder: FLAD, Michael, 88677 Markdorf (DE); HOHMANN, Sören, 76646 Bruchsal (DE); KÖPF, Florian, 76137 Karlsruhe (DE); KUHN, Elvira, 54294 Trier (DE); KUSMARTSEV, Feodor, Loughborough LE11 3JR (GB)
(74) Vertreter: Bailey Walsh & Co. LLP
(86) Internationale Anmeldenummer: PCT/EP2020/069379
(87) Internationale Veröffentlichungsnummer: WO 2021/005160

(56) Entgegenhaltungen:
- EP-A1- 3 342 390
- US-A1- 2016 346 156
- US-A1- 2019 099 113
- US-A1- 2019 343 707
- US-B2- 9 351 900

## Beschreibung

Die vorliegende Erfindung betrifft ein System zur Erkennung und Verhinderung eines bevorstehenden Sturzes eines sich bewegenden Körpers.

Jeder zwölfte Erwachsene erleidet in Deutschland mindestens einmal im Jahr einen Unfall, wobei die häufigste Unfallursache ein Sturz ist. Der Anteil beträgt fast 30 % und nimmt mit dem Alter zu, so dass bei Menschen über 70 Jahren die Mehrheit der Unfälle sturzbedingt sind. Ein nicht unerheblicher Anteil dieser Unfälle ist mit Verletzungen verbunden, die nicht selten stationär versorgt werden müssen.

Aufgrund der höheren Gefährdung durch Stürze ziehen sich ältere Menschen häufig aus dem öffentlichen Leben zurück und verbringen überwiegend Zeit in gewohnter Umgebung. Die nachlassende körperliche Leistungsfähigkeit führt somit zu zunehmender sozialer Isolation.

Es ist somit erstrebenswert, die Anzahl der Stürze zu reduzieren, ohne dabei die Alltagstauglichkeit der betroffenen Personen einzuschränken.

Selbstverständlich sind von einem erhöhten Risiko eines Sturzes nicht nur ältere Menschen betroffen, sondern auch durch andere Gründe in ihrer Leistungsfähigkeit eingeschränkte Personen, zum Beispiel während einer Rehabilitationsmaßnahme nach einem bereits erfolgten Unfall. Eine Sturzverhinderung ist ebenso sinnvoll für andere bewegliche Körper, wie zum Beispiel einem Roboter oder einem Tier.

Eine Sturzerkennung wird bisher technisch durch den Einsatz von Assistenzlösungen gelöst, die sich in der Umgebung des Menschen befinden. Beispielsweise kann ein Sturz von Sensoren unter einem Teppich, die die Lage des Menschen nach einem Sturz detektieren, erkannt werden.

Ferner gibt es Hilfsmittel, die Menschen helfen, nicht zu fallen oder gewünschte Bewegungen durchzuführen. Dazu gehören zum Beispiel Armbänder, die den Fall, wenn er bereits erfolgt ist, als solchen erkennen und einen Alarm auslösen. Zur Bewegungsunterstützung eines Menschen sind ferner Exoskelette bekannt, welche eine aktive Unterstützung der Muskeln des Trägers durchführen.

US 2016/346156 A1 betrifft ein System zur Unterstützung eines Menschen beim Erlernen des Gehens. Dabei wird der Mensch durch Einschränkung seiner Bewegungsfreiheit unterstützt.

EP 3 342 390 A1 betrifft ein System, das einen Menschen dabei unterstützt, sich in einen Rollstuhl zu setzen oder von einem Rollstuhl aufzustehen, wobei ein bevorstehender Sturz erkannt und verhindert wird.

Die genannten Systeme führen jedoch entweder erst Maßnahmen durch, wenn ein Sturz bereits erfolgt ist, oder unterstützen den Träger des Systems aktiv bei seiner momentanen Bewegung. Das heißt, die genannten Systeme sind nicht darauf ausgelegt, einen Sturz zu verhindern.

US2019099113A1 betrifft eine Methode zur Sturzerkennung.

Ferner sind Exoskelette mit aktiver Bewegungsunterstützung meist schwer, unflexibel und energieintensiv, so dass sie im Alltag keine Rolle spielen. Unflexibel bedeutet in diesem Zusammenhang, dass sie nicht leicht handhabbar von der tragenden Person angelegt werden können.. Weiterhin bedeutet es eine räumliche Gebundenheit wie oft bei Lernsystem gegeben, da diese auf das Beobachten von Trajektorien im physikalischen Sinne setzen und dazu meist optische Geräte zum Beobachten von Soll-kurven und Ist-kurven einsetzen.

Unter Beachtung der vorstehenden Nachteile oder Unzweckmäßigkeiten des Standes der Technik ist es demnach Aufgabe der vorliegenden Erfindung, ein verbessertes System und eine verbesserte Methode zur Verhinderung eines Sturzes bzw. zur unterstützenden Stabilisierung eines sich bewegenden Körpers bereitzustellen. Dabei wird unter Stabilisierung die Erhaltung eines Stabilen Zustandes eines dich bewegenden Körpers verstanden. Ohne viel Kraftaufwand von außen und in kürzester kaum wahrnehmbarer Zeit soll das Eintreten eines instabilen Zustandes verhindert werden. Die Flexibilität bei uns bezieht sich auf die Anzahl der Ansteuerung von unterschiedlich langen und dicken Aktoren, die mittels Stromimpuls ihre Biegsamkeit verlieren und hart werden. Die Ansteuerung erfolgt ausschließlich auf Grund des sich bewegenden Körpers.

Diese Aufgabe wird durch die unabhängigen Ansprüche gelöst. Die abhängigen Ansprüche definieren vorteilhafte Ausführungsformen.

Die vorliegende Erfindung betrifft ein bewegungsabhängiges Stabilisierungsunterstützungssystem zur Aufrechterhaltung der Stabilität eines sich bewegenden Körpers. Das bewegungsabhängige Stabilisierungsunterstützungssystem umfasst eine Mehrzahl an Sensoren, die ausgelegt sind, Bewegungsparameter des Körpers zu erfassen. Ferner umfasst das System eine Mehrzahl an Aktoren sowie eine Steuereinheit, die ausgelegt ist, anhand der Bewegungsparameter und eines biomechanischen Bewegungsmodells zu erkennen, ob eine Instabilität des Körpers bevorsteht. Die Steuereinheit ist ferner ausgelegt, eine Stabilisierungsstrategie aus einer Mehrzahl vorgegebener Stabilisierungsstrategien zu wählen, wenn erkannt wurde, dass eine Instabilität des Körpers bevorsteht, und die Aktoren entsprechend der gewählten Stabilisierungsstrategie anzusteuern. Dabei sind die Aktoren flexibel verformbar und versteifen bei Ansteuerung durch die Steuereinheit.

In anderen Worten betrifft die vorliegende Erfindung ein System zur Sturzverhinderung eines beweglichen Körpers, wie zum Beispiel eines Menschen oder einer Person, die sich so bewegt, dass sie im nächsten Moment ohne Eingreifen durch das System fallen würde.

Durch die Verwendung von mehreren Sensoren erfasst das System Bewegungsparameter des Körpers, wodurch die Bewegung des Körpers kontinuierlich vom System überwacht wird.

Auf Grundlage der erfassten Sensordaten bzw. der Bewegungsparameter und einem biomechanischen Bewegungsmodell wird abgeschätzt, ob der Körper in eine instabile Lage kommen wird. Anhand des Ergebnisses der Abschätzung der zukünftigen Instabilität und der Bewegungsparameter wird eine Stabilisierungsstrategie ausgewählt, nach der die Aktoren, die am Körper angebracht sind, angesteuert werden.

Eine Stabilisierungsstrategie ist dabei eine vorgegebene Vorschrift zur Ansteuerung der Aktoren für eine zugeordnete Situation bzw. geschätzte Lage des Körpers. Eine Stabilisierungsstrategie kann zum Beispiel Vorschriften enthalten, welche Aktoren zu welcher Zeit und in welcher Weise angesteuert werden sollen. Die Stabilisierungsstrategie, nach der der Körper stabilisiert werden soll, wird somit aus einer Mehrzahl vorgegebener Stabilisierungsstrategien ausgewählt. Dies erfolgt auf Grundlage der erfassten Bewegungsparameter, dem biomechanischen Bewegungsmodell und daraus bestimmten zukünftigen Lage des beweglichen Körpers.

Eine Stabilisierungsstrategie kann dabei eine zeitliche Abfolge der Ansteuerung verschiedener Aktorgruppen beinhalten, wobei eine Aktorgruppe eine vorgegebene Untermenge der Aktoren beinhaltet. Des Weiteren kann eine Stabilisierungsstrategie eine mehrstufige Ansteuervorschrift enthalten, nach der zunächst eine erste Aktorgruppe angesteuert wird, und, wenn abgeschätzt wird, dass der Sturz weiterhin bevorsteht, eine zweite Aktorgruppe angesteuert wird. Den Aktoren bzw. Aktorgruppen kann somit eine Priorität der Ansteuerung zugeordnet sein, so dass entsprechend der Auswirkung der Ansteuerung weitere Aktoren versteift werden können.

Die Aktoren sind zum Beispiel an vorgegebenen Positionen des beweglichen Körpers angebracht und sind flexibel verformbar, so dass die Bewegungsfreiheit des Trägers des Systems nicht oder nur minimal eingeschränkt wird. Bei Ansteuerung durch die Steuereinheit versteifen jedoch die Aktoren. Im Fall eines bevorstehenden Sturzes wird somit die Bewegungsfreiheit des Trägers des Systems gezielt eingeschränkt, um zu verhindern, dass die bevorstehende instabile Lage tatsächlich eintritt.

Durch die vorliegende Erfindung wird somit erreicht, dass eine bevorstehende instabile Situation des beweglichen Körpers nicht eintritt, indem eine Bewegung des Körpers in die instabile Lage unterdrückt wird. Ein bevorstehender Sturz wird somit unterbunden.

Gemäß einer spezifischen Ausführungsform ist die Steuereinheit ferner ausgelegt, einen Regelungsprozess zur Beendigung der Ansteuerung der Aktoren zur Stabilisierung basierend auf den Bewegungsparametern durchzuführen.

Das heißt, in einem Fall, in dem eine Einschränkung der Bewegung nicht mehr notwendig ist, da ein Sturz nicht mehr bevorsteht bzw. erfolgreich verhindert werden konnte, löst die Steuereinheit die Einschränkung der Bewegungsfreiheit wieder auf, indem die Ansteuerung der Aktoren im Rahmen eines Regelungsprozesses ausgesetzt wird. Zum Beispiel kann die Versteifung der einzelnen Aktoren nacheinander aufgehoben werden oder, beispielsweise, graduell verringert werden.

Dabei werden kontinuierlich weiterhin die Bewegungsparameter erfasst und im Hinblick auf eine bevorstehende Instabilität des Körpers ausgewertet. Dadurch wird sichergestellt, dass auch bei bzw. während der Beendigung der Fallverhinderung kein Sturz erfolgt. Dies wird erreicht, indem eine spezifische Regelung zur Aussetzung der Fallverhinderungsmaßnahme durchgeführt wird.

Gemäß einer bevorzugten Ausführungsform beruht das biomechanische Bewegungsmodell auf einer Formalisierung der Bewegung eines einfachen invertierten Pendels oder doppelt invertierter Pendel.

Ein invertiertes Pendel ist ein Pendel mit dem Schwerpunkt oberhalb der Achse. Ein solches Pendel befindet sich in seinem höchsten Punkt in einer Ruhelage die es jedoch schon bei kleinen Auslenkungen verlässt. Ein solches Pendel eignet sich in einfacher Weise zur Modellierung der Lage eines beweglichen Körpers, insbesondere eines Körpers menschlicher Gestalt. Ein stabiler Zustand wird üblicherweise von einer Person durch kontinuierliche Wahrnehmung der Lage und entsprechender Bewegung aufrechterhalten.

Die Nutzung eines einfachen invertierten Pendels ermöglicht die Modellierung der Lage des beweglichen Körpers in einer Weise, die nur einen minimalen Rechenaufwand benötigt.

Ein doppelt invertiertes Pendel ist eine Kombination zweier einfacher invertierten Pendel, wobei das zweite invertierten Pendel auf dem ersten invertierten Pendel angebracht ist. Ein solches doppeltes invertiertes Pendel eignet sich zur genaueren Modellierung der Lage eines menschenähnlichen Körpers. Insbesondere ist ein solches Modell in der Lage das Beugen eines beweglichen Körpers, z. B eines Körpers menschlicher Form, zu modellieren.

Gemäß einer weiteren bevorzugten Ausführungsform beruht das biomechanische Bewegungsmodell auf der Formalisierung der Bewegung eines einfachen invertierten Pendels auf einer Sprungfeder oder eines doppelten invertierten Pendels auf einer Sprungfeder.

In anderen Worten kann die Bewegung bzw. die Lage des Körpers durch ein einfaches invertiertes Pendel oder ein doppeltes investiertes Pendel, jeweils angebracht auf einer Feder, modelliert werden. Die Feder bildet dabei eine Flexibilität des zu modellieren Körpers in Richtung einer Hochachse ab.

Mit den genannten Modellen, die die Feder beinhalten, ist eine genaue bzw. wirklichkeitstreuere Modellierung der Bewegung des beweglichen Körpers möglich, wobei die Komplexität der Beschreibung der Lage bzw. der Bewegung des Körpers durch die zunehmende Anzahl der Freiheitsgrade steigt.

Gemäß einem weiteren Aspekt ist das biomechanische Bewegungsmodell ein erweitertes biomechanisches Bewegungsmodell des Körpers, das ein menschliches Körpermuskelsystem für Menschen verschiedenen Alters durch ein System invertierter Pendel, verbunden mit elastischen Federn, modelliert.

Das heißt, durch Kopplung mehrerer invertierter Pendel durch elastische Federn kann ein komplexes Modell der Bewegung des beweglichen Körpers realisiert werden. Insbesondere kann durch die Eigenschaften der Federn (zum Beispiel der Federhärte und der Länge der Federn) die Eigenschaften des beweglichen Körpers realitätsnah modelliert werden. Zum Beispiel kann die Federhärte der Koppelfedern für einen menschlichen Träger des Systems geringen Alters größer eingestellt werden als für einen menschlichen Träger höheren Alters, um die unterschiedlichen Eigenschaften des Körpermuskelsystems für Menschen verschiedenen Alters abzubilden.

Gemäß einer weiteren Ausführungsform ist die Steuereinheit ferner ausgelegt, anhand der empfangenen Bewegungsparameter des Körpers Positionen vorgegebener Referenzpunkte des biomechanischen Bewegungsmodells zu bestimmen und die bevorstehende Instabilität des Körpers zu erkennen, basierend auf Trajektorien der Referenzpunkte des biomechanischen Bewegungsmodells.

Das heißt, dass die Koordinaten (aktuelle und nächste Positionen von Referenzpunkten) des mathematischen Bewegungsmodells auf Grundlage der Bewegungsparameter des Körpers bestimmt werden. Zur Abschätzung der bevorstehenden Instabilität werden die Positionen der Referenzpunkte des durch das-mathematische Bewegungsmodell bezüglich der Lage der Unterstützungsebene ausgewertet..

Gemäß einer bevorzugten Ausführungsform erkennt die Steuereinheit die bevorstehende Instabilität des Körpers, indem anhand von Änderungen zwischen vorherigen und aktuellen Positionen der Referenzpunkte des biomechanischen Bewegungsmodells zukünftige Positionen berechnet werden. Wenn die zukünftigen Positionen zumindest einer Bedingung aus einer Mehrzahl vorgegebener Bedingungen genügen, erkennt die Steuereinheit, dass eine Instabilität des Körpers bevorsteht.

Das bedeutet, anhand von Sensordaten bzw. Bewegungsparametern, die in der Vergangenheit detektiert bzw. erfasst wurden, kann eine Voraussage für eine zukünftige Lage des Körpers getroffen werden. In anderen Worten, es wird eine Extrapolation der Lage des Körpers bzw. des biomechanischen Bewegungsmodells durchgeführt, um eine bevorstehende Instabilität des Körpers abzuschätzen.

Dazu werden die Bewegungsparameter genutzt, um Positionen von vorgegebenen Referenzpunkten des biomechanischen Bewegungsmodells zu gegebenen Zeitpunkten bzw. mit einer vorgegebenen Detektionsrate zu bestimmen. Anhand der Positionen der Referenzpunkte des Modells und den Eigenschaften des Modells (einfach/doppelt investiertes Pendel, Federhärte, Längen des Pendels, ...) wird die Lage in die Zukunft extrapoliert und anhand der zukünftigen Positionen die Stabilität bzw. Instabilität abgeschätzt.

Gemäß einem weiteren Aspekt umfassen die Sensoren jeweils eine Speichereinheit und eine Recheneinheit, wobei die Speichereinheit ausgelegt ist, vorherige Sensordaten zu speichern und die Recheneinheit ausgelegt ist, die vorherigen und aktuellen Sensordaten zu verarbeiten und ein Ergebnis der Verarbeitung als Bewegungsparameter des Körpers zu erfassen.

Das heißt, dass Primärsensordaten zunächst innerhalb des Sensors selbst vorprozessiert werden und das Ergebnis als Bewegungsparameter an die Steuereinheit weitergegeben wird. Zum Beispiel kann die Vorprozessierung eine Bestimmung der Veränderungen der Primärsensordaten beinhalten, so dass eine Veränderung des entsprechenden Messwerts anstatt des Messwerts selbst der Steuereinheit als Bewegungsparameter zur Verfügung gestellt wird. Dadurch wird der Rechenaufwand der Steuereinheit reduziert und zusätzlich die zu übertragende Datenmenge verringert.

Nach einer bevorzugten Ausführungsform erkennt die Steuereinheit nach Erkennen der bevorstehenden Instabilität des Körpers anhand von Bewegungsparametern des Körpers, ob die Instabilität des Körpers weiterhin vorliegt oder nicht. Die Steuereinheit steuert die Aktoren entsprechend an.

Das heißt, dass nach Erkennung der bevorstehenden Instabilität nicht nur die entsprechende Maßnahme getroffen wird (Auswahl der Stabilisierungsstrategie und Ansteuerung der entsprechenden Aktoren), sondern kontinuierlich die Lage bzw. Bewegung des Körpers überwacht wird, so dass erkannt werden kann, ob die Instabilität weiterhin bevorsteht oder die eingeleitete Stabilisierungsstrategie bzw. Fallverhinderung beendet werden kann.

Gemäß einer spezifischen Ausführungsform weisen die Aktoren eine schlauchähnliche Form auf und sind mit einem magnetisch-sensitiven oder elektrisch-sensitiven Medium gefüllt, das bei Ansteuerung durch die Steuereinheit versteift.

In anderen Worten, die Aktoren weisen eine längliche Form mit im Vergleich zur Länge geringer Breite auf. Die äußere Struktur der Aktoren ist dabei hohl und mit einem Material gefüllt, das bei Anlegen eines elektrischen oder magnetischen Felds seine elastische Verformbarkeit ändert, so dass ein höherer Kraftaufwand notwendig ist, um die gleiche Verformung zu erreichen, im Vergleich zu dem Fall, dass kein elektrisches oder magnetisches Feld angelegt ist. Alternativ kann das genannte Material seine Verformbarkeit ändern, wenn ein Stromfluss durch das Material vorhanden ist.

Dadurch wird erreicht, dass bei Ansteuerung der Aktoren durch die Steuereinheit die Aktoren versteifen, wodurch die Bewegungsfreiheit des Körpers eingeschränkt wird. Im Gegensatz, wenn kein elektrisches bzw. magnetisches Feld angelegt ist, sind die Aktoren durch minimalen Kraftaufwand verformbar, so dass die Bewegungsfreiheit des Trägers des Systems nicht oder nur minimal eingeschränkt ist.

Beispielsweise handelt es sich bei einem elektrisch-sensitiven Material um elektroaktive Polymere, die durch das Anlegen einer elektrischen Spannung ihre mechanischen Eigenschaften ändern.

In einer spezifischen Ausführungsform beinhalten die Aktoren eine magnetorheologische Flüssigkeit, die bei Anlegen eines Magnetfeldes versteift. In diesem Fall umfassen die Aktoren jeweils einen Magnetfeldgenerator, der bei Ansteuerung durch die Steuereinheit ein Magnetfeld zur Versteifung der magnetorheologischen Flüssigkeit erzeugt.

Bei einer magnetorheologischen Flüssigkeit handelt es sich um eine Suspension magnetisch polarisierter Partikel, die in einer Trägerflüssigkeit fein verteilt sind. Dabei sind die Partikel um ca. ein bis drei Zehnerpotenzen größer als die der Ferrofluide, wodurch sich eine magnetorheologische Flüssigkeit beim Anlegen eines Magnetfeldes verfestigt.

Nach einem weiteren Aspekt sind die Sensoren inertiale Messeinheiten, die Beschleunigungen oder Drehraten als Bewegungsparameter des Körpers erfassen.

Zum Beispiel kann es sich dabei um Sensoren zur Messung einer linearen Beschleunigung, der Änderung einer Drehrate oder einer Drehrate selbst handeln. Durch einfache oder mehrfache Integration der Messwerte in der Zeitdimension kann auf Positionen bzw. Winkel geschlossen werden.

Der sich bewegenden Körper kann eine Person oder ein Roboter menschlicher Gestalt sein.

Gemäß einer spezifischen Ausführungsform sind die Mehrzahl der Sensoren ausgelegt, zumindest an Hüften, Schultern, Händen und/oder Kopf der Person oder des Roboters menschlicher Gestalt angebracht zu werden.

Das bedeutet, durch die Auswertung der Sensordaten können Positionen der Hüften, Schultern, Armen, Händen und/oder des Kopfes ermittelt werden.

Nach einem weiteren Aspekt sind die Mehrzahl Aktoren ausgelegt, im Bereich von Gelenken und/oder des Torsos der Person oder des Roboters menschliche Gestalt angebracht zu werden, um die Bewegungsfreiheit der Gelenke und/oder des Torsos durch versteifen bei Ansteuerung durch die Steuereinheit einzuschränken.

Gemäß einer weiteren spezifischen Ausführungsform umfasst das bewegungsabhängige Stabilisierungsunterstützungssystem ferner eine Mehrzahl zweiter Aktoren, die ausgelegt sind, bei Ansteuerung durch die Steuereinheit eine aktive Bewegung durchzuführen. Dabei ist die Steuereinheit ferner ausgelegt, die zweiten Aktoren entsprechend der gewählten Stabilisierungsstrategie anzusteuern.

Das heißt, zusätzlich zu den Aktoren, die bei Ansteuerung durch die Steuereinheit versteifen, kann das System weitere zweite Aktoren umfassen, die bei Ansteuerung durch die Steuereinheit eine aktive Bewegung durchführen.

Damit kann zusätzlich zur Einschränkung der Bewegungsfreiheit des Körpers auch aktiv in die Bewegung eingegriffen werden, so dass, wenn ein Sturz bevorsteht und dieser allein durch Versteifen der Aktoren nicht verhindert werden kann, eine Bewegung des Körpers in Richtung eines stabilen Zustands durch die zweiten Aktoren erzwungen werden kann.

Nach einem weiteren Aspekt kann das bewegungsabhängige Stabilisierungsunterstützungssystem eine Speichereinheit umfassen, die erfasste Bewegungsparameter sowie ausgewählte und durchgeführte Stabilisierungsstrategien kontinuierlich speichert.

Zudem kann das System eine Programmierschnittstelle (API) umfassen, über die der gespeicherte Inhalt der Speichereinheit einem externen Gerät zur Verfügung gestellt werden kann.

Auf Grundlage der zur Verfügung gestellten Parameter und der gewählten/durchgeführten Stabilisierungsstrategien kann eine Analyse der Effekte der Stabilisierungsstrategien durchgeführt werden, um die Parameter des biomechanischen Bewegungsmodells sowie die Stabilisierungsstrategien anzupassen, wodurch die Stabilisierungsunterstützung optimiert werden kann.

Dadurch, wie auch bereits durch einen Initialisierungsvorgang, kann das System und das verwendete Modell an die individuellen Eigenschaften des Trägers angepasst werden.

Die vorliegende Erfindung betrifft ferner ein bewegungsabhängiges Stabilisierungsunterstützungsverfahren zur Aufrechterhaltung der Stabilität eines sich bewegenden Körpers. Das bewegungsabhängige Stabilisierungsunterstützungsverfahren umfasst das Erfassen von Bewegungsparametern des Körpers und das Erkennen, ob eine Instabilität des Körpers bevorsteht, anhand der Bewegungsparameter und eines biomechanischen Bewegungsmodells. Ferner umfasst das Verfahren das Auswählen einer Stabilisierungsstrategie aus einer Mehrzahl vorgegebener Stabilisierungsstrategien, wenn erkannt wurde, dass eine Instabilität des Körpers bevorsteht, sowie das Versteifen von flexibel verformbaren Aktoren entsprechend der gewählten Stabilisierungsstrategie.

Mit dem System der vorliegenden Erfindung kann ein zukünftiger instabiler Zustand des Trägers des Systems, d.h., des sich bewegenden Körpers, zuverlässig vorausgesagt werden und entsprechende Gegenmaßnahmen ergriffen werden. Insbesondere kann die Bewegungsfreiheit des Körpers dahingehend eingeschränkt werden, dass eine Bewegung, die in einen instabilen Zustand führen würde, erschwert wird. Dadurch wird erreicht, dass der bevorstehende Sturz nicht stattfindet.

Zusätzliche Vorteile und Vorzüge der vorliegenden Erfindung ergeben sich aus der detaillierten Beschreibung einer bevorzugten Ausführungsform und den Zeichnungen.

### KURZE BESCHREIBUNG DER ZEICHNUNGEN

Fig. 1 zeigt ein schematisches Blockschaltbild der Komponenten des bewegungsabhängigen Stabilisierungsunterstützungssystems.
Fig. 2 zeigt die Lage der Aktoren in einer Ausführungsform des bewegungsabhängigen Stabilisierungsunterstützungssystems an einem menschlichen Träger.
Fig. 3 zeigt die Lage der Sensoren und der Steuereinheit in einer Ausführungsform des bewegungsabhängigen Stabilisierungsunterstützungssystems an einem menschlichen Träger.
Fig. 4A bis 4D zeigen exemplarische biomechanische Bewegungsmodelle, anhand derer die zukünftige Stabilität eines Trägers des Stabilisierungsunterstützungssystems ermittelt werden kann.
Fig. 5A bis 5C zeigen beispielhaft die Lage des Schwerpunkts eines Trägers des Stabilisierungsunterstützungssystems und dessen vertikale Position auf die Standebene des Körpers.
Fig. 6A zeigt beispielhaft die Lage der vertikalen Projektion des Schwerpunkts auf die Standebene des Körpers und dessen relative Position zur Unterstützungsbasis im Fall einer stabilen Lage des Körpers.
Fig. 6B zeigt beispielhaft die Lage der vertikalen Projektion des Schwerpunkts auf die Standebene des Körpers und dessen relative Position zur Unterstützungsbasis im Fall einer instabilen Lage des Körpers.
Fig. 7A bis 7E zeigt beispielhaft die Lage der Unterstützungsbasis für verschieden Stellungen der Füße eines menschlichen Trägers des Systems im Stand und in Bewegung.
Fig. 8 zeigt beispielhaft eine Formalisierung einer Bewegung als Trajektorie eines doppelt invertierten Pendels auf einer Sprungfeder.
Fig. 9 zeigt ein Ablaufdiagramm eines Verfahrens zur Stabilisierungsunterstützung eines sich bewegenden Körpers.
Fig. 10 zeigt ein schematisches Blockschaltbild eines Sensors (MEM-SSC), der vorprozessierte Sensordaten als Bewegungsparameter an die Steuereinheit weiterleitet.
Fig. 11 zeigt beispielhaft eine Ausführungsform des Stabilisierungsunterstützungssystems, das zusätzlich zweite Aktoren, die eine aktive Bewegung durchführen, umfasst.

### AUSFÜHRLICHE BESCHREIBUNG DER BEVORZUGTEN AUSFÜHRUNGSFORM

Im Folgenden wird eine bevorzugte Ausführungsform der vorliegenden Erfindung anhand der Zeichnungen detailliert beschrieben.

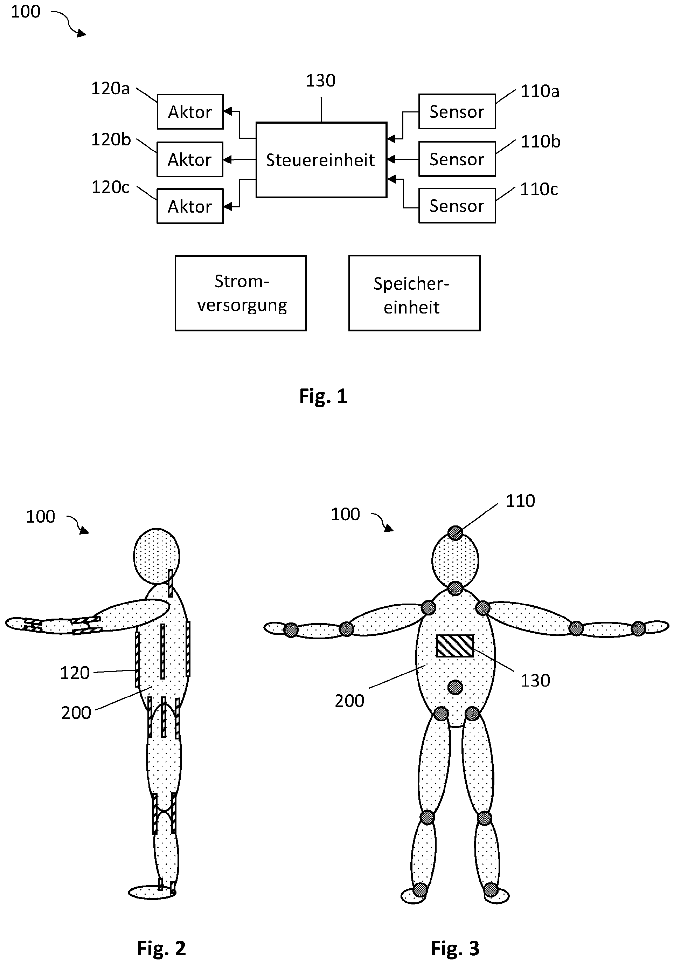

Fig. 1 zeigt ein Blockschaltbild des bewegungsabhängigen Stabilisierungssystems 100 nach einer bevorzugten Ausführungsform.

Das bewegungsabhängige Stabilisierungssystem 100 (im Folgenden auch als Stabilisierungssystem 100 oder System 100 bezeichnet) umfasst eine Mehrzahl an Sensoren 110a-110c, die Bewegungsparameter des Körpers 200 erfassen. Ferner umfasst das System eine Mehrzahl an Aktoren 120a-120c sowie eine Steuereinheit 130. Zusätzlich beinhaltet das System eine Stromversorgung, beispielsweise eine Batterie, einen Akkumulator, ein System zur kabellosen Stromversorgung oder eine andere Energieversorgungsquelle, zur Energieversorgung der einzelnen Komponenten sowie eine Speichereinheit wie zum Beispiel ein RAM, ein ROM oder ein Festplattenlaufwerk, auf die die Steuereinheit 130 zugreifen kann.

Figuren 2 und 3 zeigen die Lage der Sensoren 110 und Aktoren 120 des Stabilisierungssystems 100 an einem menschlichen Träger (einer Person) als beweglicher Körper 200. Die Aktoren 120 sind in den Bereichen der Gelenke der Person 200 angebracht, sowie im Bereich des Torsos auf der Vorderseite, auf der Rückseite (dem Rücken) sowie seitlich. Ferner ist ein Aktor 120 im Bereich des Nackens der Person befestigt.

Die Sensoren 110 befinden sich an der Oberseite des Kopfes, im Bereich des Halses sowie nahe der Körpermitte und der Gelenke im Bereich der Schultern, der Ellenbogen, der Handgelenke, der Hüften, der Knie sowie der Knöchel.

Die vorliegende Erfindung ist nicht auf die beschriebene Lage der Aktoren 120 beschränkt. Beispielsweise können Aktoren 120 nur im Bereich der Beine und der Hüfte angebracht sein. Ferner können sich im Bereich des Rückens, insbesondere im Bereich der Wirbelsäule eine Mehrzahl an Aktoren 120 befinden.

Ferner ist die vorliegende Erfindung nicht auf die beschriebene Lage der Sensoren 110 beschränkt. Beispielsweise können diese anstatt im Bereich der Gelenke der Person 200 in der Mitte der Extremitätenteile angebracht sein. So könnte jeweils ein Sensor im Bereich der Handfläche, im Bereich der Unter- und Oberarme sowie im Bereich der Unter- und Oberschenkel angebracht sein.

Ferner umfasst das Stabilisierungssystem 100 die Steuereinheit 130, die, wie aus Fig. 3 ersichtlich, im Bereich des Brustkorbs der Person 200 angebracht ist. Die vorliegende Erfindung ist jedoch nicht auf diese Lage der Steuereinheit beschränkt. Vielmehr kann diese an einer beliebigen Stelle des Körpers 200 angebracht sein. Alternativ kann sie nicht direkt an der Person befestigt, sondern zum Beispiel in einem Rucksack getragen werden oder auf einem integrierten Schaltkreis (Chip) befindlich injiziert sein.

Das Stabilisierungssystem umfasst ferner eine Stromversorgung bzw. Energieversorgung, wie zum Beispiel eine Batterie zusammen mit Leitungen zur Spanungsversorgung, sowie Kommunikationsleitungen zur Signalübertragung zwischen den Einzelkomponenten des Systems 100.

Die vorliegende Erfindung ist nicht auf ein zentrales Energieversorgungssystem beschränkt. Vielmehr kann jede Einzelkomponente eine entsprechende Energieversorgung beinhalten.

Diese kann zum Beispiel als gemeinsame Übertragungsleitung zwischen den Einzelkomponenten des Stabilisierungssystems 100 in Form einer Bus-Leitung realisiert sein. Die vorliegende Erfindung ist jedoch nicht auf eine Datenübertragung in einem Bus-System beschränkt, vielmehr kann die Datenübertragung zwischen den Einzelkomponenten auf beliebige Weise erfolgen. Auch eine Kabellose Datenübertragung zwischen den Komponenten, beispielsweise über ein lokales drahtloses Netzwerk (WLAN) oder über Bluetooth^{®}-Verbindungen, ist möglich.

Das System 100 kann in eine Trägerkonstruktion, die an der Person 200 befestigt werden kann, eingebettet sein. Alternativ können die Komponenten in Textilien, die von der Person 200 getragen werden, integriert sein. Die vorliegende Erfindung ist nicht auf ein spezifisches Trägersystem oder Methode der Befestigung der Einzelkomponenten beschränkt, vorausgesetzt, dass die Sensoren benötigte Bewegungsparameter erfassen können und die Aktoren bei Versteifen die Bewegungseinheit der Person 200 einschränken.

Die Sensoren 110 erfassen Bewegungsparameter des Körpers 200 und leiten diese an die Steuereinheit 130 weiter. Bei den Bewegungsparametern kann es sich zum Beispiel um Beschleunigungswerte, Geschwindigkeitswerte oder Drehraten handeln, je nach Ausprägung der einzelnen Sensoren 110a-110c. Die Übertragung der Bewegungsparameter erfolgt dabei in einer vorgegebenen Regelmäßigkeit, zum Beispiel alle 0.1, 0.2, 0.5 oder 1 Sekunde.

Die vorliegende Erfindung ist jedoch nicht auf einen spezifischen Übertragungs- /Erfassungstakt beschränkt. Vielmehr können die Bewegungsparameter zu beliebigen regelmäßigen oder auch unregelmäßigen Zeiten an die Steuereinheit 130 übertragen werden.

Die Steuereinheit umfasst ferner eine Speichereinheit, die die empfangenen Bewegungsparameter speichert.

Anhand der empfangenen Bewegungsparameter oder Veränderungen der Bewegungsdaten ermittelt die Steuereinheit 130 Positionen von Referenzpunkten eines biomechanischen Bewegungsmodells.

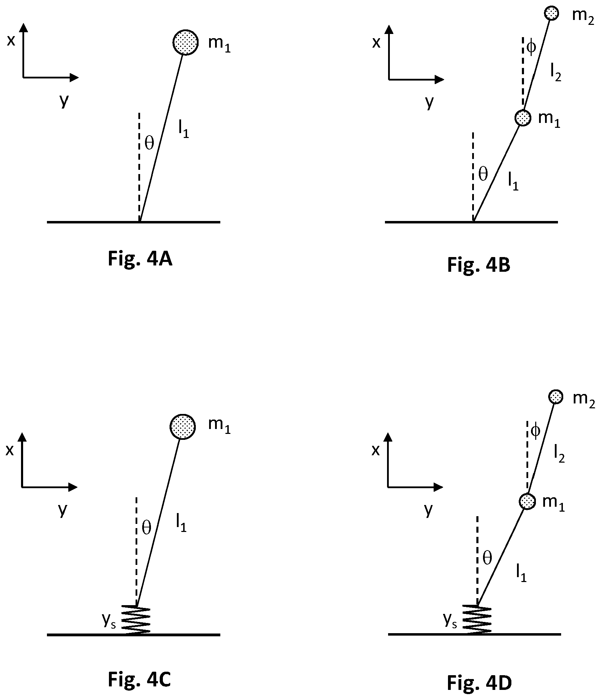

Figuren 4A bis 4B zeigen beispielhaft vier biomechanische Bewegungsmodelle unterschiedlicher Komplexität.

In Fig. 4A ist beispielhaft ein biomechanisches Bewegungsmodell, das auf einem einfachen invertierten Pendel beruht, dargestellt. Die Lage des biomechanischen Bewegungsmodells ist dabei durch die Winkelauslenkung θ aus der Gleichgewichtsposition eindeutig beschrieben. Die Parameter l₁ sowie m₁, die die Länge des Pendels und die Masse bezeichnen, sind vorgegeben und können zum Beispiel im Rahmen einer Initialisierung des Systems 100 konfiguriert werden.

In Fig. 4B ist beispielhaft ein biomechanisches Bewegungsmodell, das auf doppelt invertierten Pendeln beruht. Die Lage dieses biomechanischen Bewegungsmodells ist durch die Winkelauslenkung θ des ersten invertierten Pendels sowie die Winkelauslenkung φ des zweiten invertierten Pendels beschrieben. Die Parameter l₁, m₁, l₂ und m₂, die die Länge des ersten Pendels, die erste Masse, die Länge des zweiten Pendels und die zweite Masse bezeichnen, sind vorgegeben und können, wie für das einfache invertierte Pendel im Rahmen einer Initialisierung des Systems 100 konfiguriert werden.

Die in den Figuren 4C und 4D dargestellten beispielhaften biomechanischen Bewegungsmodelle entsprechen den in den Figuren 4A und 4B gezeigten Modellen, wobei die Modelle zusätzlich eine Sprungfeder enthalten, auf der die Pendel angebracht sind. Als weitere Parameter der Lage der Bewegungsmodelle kommt damit die Federauslenkung yₛ sowie die Federhärte k der Feder hinzu.

Wie bereits beschrieben, können die Parameter des biomechanischen Bewegungsmodells, die die Eigenschaften des Modells beschreiben, im Rahmen einer Initialisierung des Systems 100 konfiguriert werden. Dabei können die Eigenschaften des Körpers 200, der durch das System 100 stabilisiert werden soll, berücksichtigt werden, so dass das Modell die Bewegung der Person (des Körpers) 200 optimal abbilden kann.

Zum Beispiel können die Eigenschaften der Person 200, auf deren Grundlage die Modellparameter initialisiert werden, die Körpergröße, die Länge der Extremitäten, das Alter und das Gewicht umfassen. Die vorliegende Erfindung ist jedoch nicht auf diese Eigenschaften beschränkt. Insbesondere kann eine Standard-Konfiguration vorgegeben sein, die die individuellen geometrischen Eigenschaften des Körpers nicht berücksichtigt. Andererseits kann als Grundlage der Initialisierung der Modellparameter zum Beispiel eine detaillierte Gewichtsverteilung und/oder Geometrie der Person 200 dienen.

Die in den Figuren 4A bis 4D dargestellten biomechanischen Bewegungsmodelle sind auf zwei Dimensionen (x, y) beschränkt, wobei mit y eine Vorwärtsrichtung des Körpers 200 und mit y eine Hochachse des Körpers 200 bezeichnet ist. Die vorliegende Erfindung ist jedoch nicht auf ein Modell in zwei Dimensionen beschränkt und eine Erweiterung der biomechanischen Bewegungsmodelle auf drei Dimensionen, die zusätzlich eine Auslenkung senkrecht zu den dargestellten Richtungen, d. h., in die bzw. aus der Zeichenebene, berücksichtigt, ist möglich. Zur Beschreibung der Lage der Bewegungsmodelle werden dann zusätzliche Winkelauslenkungskoordinaten benötigt, die die Auslenkung der einzelnen Pendel in die Zeichenebene abbilden. Da eine Erweiterung auf drei Dimensionen das Grundprinzip der vorliegenden Erfindung nicht ändert, sondern durch den höheren Komplexitätsgrad eine genauere/erweiterte Beschreibung der Lage des Körpers 200 ermöglichen, wurde auf bildliche Darstellung an dieser Stelle verzichtet.

Ferner ist die vorliegende Erfindung nicht auf die in den Figuren 4A bis 4D dargestellten Bewegungsmodelle beschränkt. Vielmehr können unter Hinzunahme weiterer Pendel zum Beispiel die Lage der Arme, beider Beine und/oder des Kopfes berücksichtigt werden. Ferner kann das biomechanische Bewegungsmodell ein weiteres invertiertes Doppelpendel beinhalten, das mit dem dargestellten Modell über eine weitere Feder mit zugeordneter Federhärte gekoppelt ist.

Auf Basis der Bewegungsparameter, die von den Sensoren 110 erfasst und an die Steuereinheit 130 weitergeleitet werden, ermittelt die Steuereinheit 130 zunächst die Koordinaten zur Beschreibung der Lage des biomechanischen Bewegungsmodells. Ferner ermittelt die Steuereinheit auf Grundlage der Bewegungsparameter und der Lage des biomechanischen Bewegungsmodells die Position von Referenzpunkten, insbesondere die Position des Kopfes, der Hände, des Körperschwerpunkt und/oder der Füße der Person 200. Ferner ermittelt die Steuereinheit auf Grundlage der Bewegungsparameter und der Lage des biomechanischen Bewegungsmodells die Position von Referenzpunkten, insbesondere die Position des Kopfes, der Hände, des Körperschwerpunkt und/oder der Füße der Person 200. Ferner ermittelt die Steuereinheit 120 die Position der vertikalen Projektionen der Referenzpunkte auf die Standebene des Körpers.

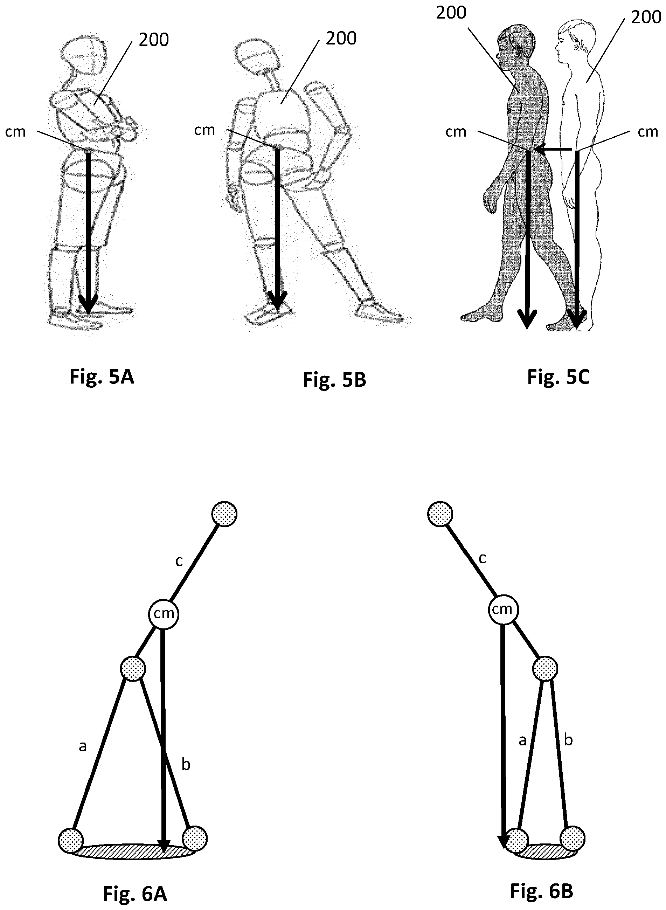

Die Figuren 5A bis 5C zeigen beispielhaft die vertikale Projektion des Körperschwerpunkts CM auf die Standebene der Person 200. Figuren 5A und 5B zeigen die Person 200 im Stand in unterschiedlichen Lagen des Körpers und die entsprechenden vertikalen Projektionsrichtungen den Körperschwerpunkts CM. Fig. 5C zeigt die Position des Körperschwerpunkts CM einer Person 200 in der Bewegung im Vergleich zum Stand. Die Bewegungsrichtung ist als horizontaler Pfeil angedeutet.

Die Figuren 6A und 6B zeigen schematische Lagen biomechanischer Bewegungsmodelle, die jeweils die beiden Beine (a, b) sowie den Oberkörper (c) einer Person 200 abbilden. Die vertikale Projektion des Körperschwerpunks CM auf die Standebene ist durch einen Pfeil gekennzeichnet. Durch die Positionen der Füße ist die Unterstützungsbasis definiert, die durch eine Fläche zwischen den Kontaktpunkten der Füße mit der Standebene des Körpers 200 beschrieben ist.

In Fig. 6A befindet sich die Projektion des Körperschwerpunkts CM innerhalb der Unterstützungsbasis. Eine solche Lage des Bewegungsmodells in Fig. 6A kann als "stabil" angesehen werden. Im Gegensatz dazu befindet sich die Projektion des Körperschwerpunkts des Bewegungsmodells in Fig. 6B außerhalb der Unterstützungsbasis, so dass die Lage des Bewegungsmodells als "instabil" angesehen werden kann.

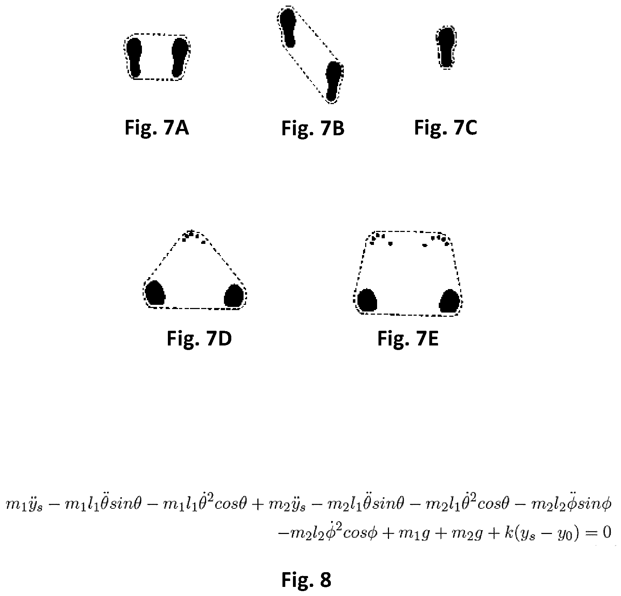

Zur Verdeutlichung der Definition der Unterstützungsbasis zeigen die Figuren 7A bis 7C die Kontaktflächen der Füße einer Person 200 mit der Standfläche sowie die dadurch definierte Unterstützungsbasis als Fläche zwischen den und einschließlich der Kontaktflächen der Füße für eine Person 200 im Stand. Die Unterstützungsbasis ist durch eine gestrichelte Linie gekennzeichnet. Die Figuren 7A und 7B zeigen einen zweibeinigen Stand, wohingegen Fig. 7C einen einbeinigen Stand darstellt.

Die Figuren 7D und 7E zeigen jeweils eine Unterstützungsbasis für eine Person 200 in Bewegung. Aufgrund der Bewegung der Person 200 ist die Unterstützungsbasis im Vergleich zum Stand nach vorne verlängert.

Die Steuereinheit 130 ermittelt auf Basis der Bewegungsparameter zu bestimmten Zeitpunkten die Positionen von Referenzpunkten der Person 200 sowie die entsprechende Lage mit Hilfe des biomechanischen Bewegungsmodells. Die Zeitpunkte können zum Beispiel durch ein zeitlich äquidistantes Raster definiert sein. Das bedeutet, dass die Bestimmung der Positionen der Referenzpunkte und die Lage des Bewegungsmodells in gewissen Zeitschritten ermittelt wird. Anhand der vorangegangen Bewegungsparameter, Referenzpositionen und der Lage des Bewegungsmodells, zum Beispiel zu einem Zeitpunkt tᵢ₋₁ sowie zum momentanen Zeitpunkt tᵢ wird die Lage des Bewegungsmodells zu einem zukünftigen Zeitpunkt tᵢ₊₁ anhand des Bewegungsmodells ermittelt.

Dies erfolgt auf Basis einer Formalisierung der Bewegung des Bewegungsmodells, wie beispielhaft in Fig. 8 dargestellt. Fig. 8 zeigt die Formalisierung eines Bewegungsmodells eines doppelt invertierten Pendels auf einer Sprungfeder, wie in Fig. 4D dargestellt. Anhand des mathematischen Modells wird die zukünftige Lage durch die Berechnung der Positionen des COM des Körpers oder Referenzpunkte der Körperteile, die Winkel zum Lot und Projektion auf die Unterstützungsbasis sowie die Unterstützungsbasis selbst durch entsprechende Referenzpositionen der Person 200 berechnet.

Nach Berechnung der zukünftigen Lage des Bewegungsmodells ermittelt die Steuereinheit 130, ob die zukünftige Lage einer vorgegebenen Bedingung genügt. Die vorgegebenen Bedingungen können zum Beispiel in Form einer Datenbank auf der Speichereinheit abgelegt sein, auf die die Steuereinheit 130 zugreift. Die vorgegebenen Bedingungen definieren Lagen des Bewegungsmodells bzw. Positionen der Referenzpunkte des Körpers, die mit einer Instabilität der Person 200 assoziiert sind und ein Eingreifen des Systems 100 erfordern, um einen bevorstehenden Sturz zu verhindern.

Dazu ist jede vorgegebene Bedingung mit einer Stabilisierungsstrategie verknüpft. Eine Stabilisierungsstrategie stellt dabei eine Vorschrift zur Ansteuerung der Aktoren 120 dar, um die Person 200 davor zu bewahren, in die bevorstehende instabile Lage zu kommen. Das heißt, auf Grundlage der bestimmten zukünftigen Lage des Bewegungsmodells und der entsprechenden Referenzpositionen der Person 200 wird eine entsprechende Stabilisierungsstrategie gewählt, wenn abgeschätzt wurde, dass die Person 200 (der Körper) in eine instabile Lage kommen wird.

Die Stabilisierungsstrategien definieren dabei die anzusteuernden Aktoren sowie die Zeit deren Ansteuerung. Dabei kann zum Beispiel eine Prioritätensteuerung definiert sein. Das heißt, dass zunächst ein bestimmter Aktor 120 oder eine bestimmte Gruppe von Aktoren angesteuert wird, und nur wenn in einem nächsten Zeitschritt abgeschätzt wird, dass durch die Ansteuerung der Aktoren 120 die zukünftige instabile Lage der Person 200 nicht verhindert werden konnte, ein zweiter Aktor oder eine zweite Aktorgruppe angesteuert wird.

Die Steuereinheit 130 steuert die Aktoren 120 entsprechend der gewählten Stabilisierungsstrategie an. Während der Ansteuerung der Aktoren 120, d.h. während der Durchführung der Stabilisierungsstrategie entsprechend der gewählten Stabilisierungsstrategie, wird weiterhin kontinuierlich die momentane und zukünftige Lage der Person 200 bestimmt und, wenn die bevorstehende Instabilität verhindert wurde, wird die Ansteuerung der Aktoren 120 beendet. Dabei wird ein Regelungsprozess zur Beendigung der Ansteuerung der Aktoren von der Steuereinheit 130 durchgeführt.

Zur Stabilitätsanalyse können beispielsweise folgende Geometriedaten genutzt werden: Körpergröße, Gewicht der Person 200, Länge der Beine und der Arme, Höhe des Körperzentrums über dem Boden und seine jeweilige Position als Lot auf die Standfläche projiziert, die Unterstützungsbasis, die Kopfzentrumsposition und/oder die Positionen von Gelenken wir Knöchel, Knie, Hüften, Schultern, Ellenboden oder Handgelenke. Referenzpunkte sind die Punkte am Körper, die durch Projektion auf die Grundebene den Bereich bestimmen, innerhalb dessen sich die Schwerpunkte eines Körpers befinden müssen um stabil zu bleiben (siehe auch Fig 6 A-6 B, und Fig 7A - 7 E) oder Punkte, die nur in einer bestimmten Range sich verändern dürfen (siehe Tab.1), um den Körper stabil zu halten. Sie sind also stabilitätsbestimmend

Die folgende Tabelle 1 zeigt beispielhaft einige vorgegebene Bedingungen zur Ermittlung einer bevorstehenden Instabilität der Person 200.

**Tabelle 1: Beispielhafte vorgegebene Bedingungen zur Ermittlung einer bevorstehenden Instabilität der Person 200.**

| **Position** | **Bereich/cm** | **Änderung/cm** |
|---|---|---|
| Körperzentrum | 10-40 | 5-15 |
| Hüfte rechts | 10-15 | 5-10 |
| Schulter rechts | 10-60 | 5-20 |
| Schulter links | 10-60 | 5-20 |
| Abstand Körperzentrum-Unterstützungsbasis | 5-35 | 5-20 |
| ... | ... | ... |

In Tabelle 1 sind unter dem Spaltennamen Position die Referenzpunkte der Person gelistet. Der Bereich gibt einen zulässigen Bereich des Referenzpunktes relativ zur entsprechenden Ruhelage an. In Tabelle 1 ist jeweils eine Wertespanne für den Bereich angegeben (z.B. 10-40 cm für das Körperzentrum). Dies spiegelt wider, dass der zulässige Bereich individuell für jeden Träger innerhalb der angegebenen Spanne festgelegt werden kann, z.B. abhängig von Körpergröße, Gewicht, Alter oder anderen Eigenschaften der Person 200.

Wird von der Steuereinheit 130 ermittelt, dass eine zukünftige Position eines Referenzpunkts, beispielsweise des Körperzentrums, außerhalb des spezifizierten Bereichs liegt, wird eine entsprechende Stabilisierungsstrategie ausgeführt.

Die erwartete Änderung in Tabelle 1 gibt eine zulässige Änderung der Position des Referenzpunktes innerhalb des zulässigen Bereichs an. In gleicher Weise wie für den zulässigen Bereich ist auch hier eine Wertespanne angegeben, innerhalb derer die zulässige Änderung individuell für den Träger des Systems 100 eingestellt werden kann. Liegt die abgeschätzte Änderung der Position eines Referenzpunktes über der erwarteten (zulässigen) Änderung, so wird eine entsprechende Stabilisierungsstrategie ausgewählt und durchgeführt.

Das bedeutet, es wird nicht nur überwacht, ob die zukünftige Position innerhalb des zulässigen Bereichs liegt, sondern auch, ob die erwartete Änderung der Position eine zulässige Änderung überschreitet. In beiden Fällen wird eine Stabilisierungsstrategie gewählt und die Aktoren 120 entsprechend angesteuert.

Alternativ oder zusätzlich zur Überwachung der Positionen der Referenzpunkte kann auch die Geschwindigkeit, Beschleunigung, Winkel oder Drehrate der Referenzpunkte überwacht werden. Überschreiten diese Parameter einen vorbestimmten Schwellenwert, wird eine Stabilisierungsstrategie gewählt und ausgeführt. Beispielsweise kann eine zukünftige Instabilität erkannt werden, wenn die Beschleunigung, die am Rücken der Person 200 gemessen wird, einen Wert von 2 cm/s² überschreitet.

Ferner können anstelle oder zusätzlich zu den Positionen der Referenzpunkte Drehraten oder Winkel überwacht werden und mit entsprechenden Stabilisierungsstrategien verknüpft sein.

Die vorliegende Erfindung ist nicht auf die oben beschriebenen vorbestimmten Bedingungen beschränkt. Vielmehr können weitere Bedingungen definiert sein, die einen zulässigen Bereich oder eine zulässige Änderung einer Position, Geschwindigkeit oder Beschleunigung eines Referenzpunkts des Körpers 200 definieren.

Ermittelt die Steuereinheit 130, dass eine Instabilität des Körpers bevorsteht, unter Nutzung der vorgegebenen Bedingungen, wird eine entsprechende Stabilisierungsstrategie ausgewählt und die Aktoren 120 entsprechend aktiviert.

Mögliche Stabilisierungsstrategien, die mit den Definitionen einer bevorstehenden Instabilität verknüpft sind, können sein: Stützung von Rücken und Nacken, Versteifen der Arme, um unkontrollierte Bewegungen zu verringern, oder Versteifung von Hüfte, Knien und Fußgelenken zu Stabilisierung.

Beispielsweise wird bei Vornüberkippen einer Person 200 (Kopf und Hände außerhalb der zulässigen Bereiche) eine Stabilisierungsstrategie gewählt, bei der Arm- und Handgelenke durch Ansteuern entsprechender Aktoren versteift werden, um ein Rudern oder Drehen der Arme bzw. Hände zu verhindern. Ferner wird der Rücken versteift.

Als weiteres Beispiel wird bei zu schneller Bewegung einer sich setzenden Person 200 (erkannt z.B. durch eine Geschwindigkeit der Hüfte und Winkelgeschwindigkeit der Kniegelenke außerhalb des jeweils zulässigen Bereichs) die Bewegung durch Aktivierung der Aktoren 120, die an den Kniegelenken angebracht sind, verlangsamt.

Die vorliegende Erfindung ist jedoch nicht beschränkt auf die beschriebenen Stabilisierungsstrategien. Vielmehr stellt eine Stabilisierungsstrategie, die mit einer vorgegebenen Bedingung verknüpft ist, eine Ansteuervorschrift zur Ansteuerung der Aktoren 120 dar. Diese Vorschrift beinhaltet, welche Aktoren zu welcher Zeit angesteuert werden, sollte die entsprechende Bedingung erfüllt sein.

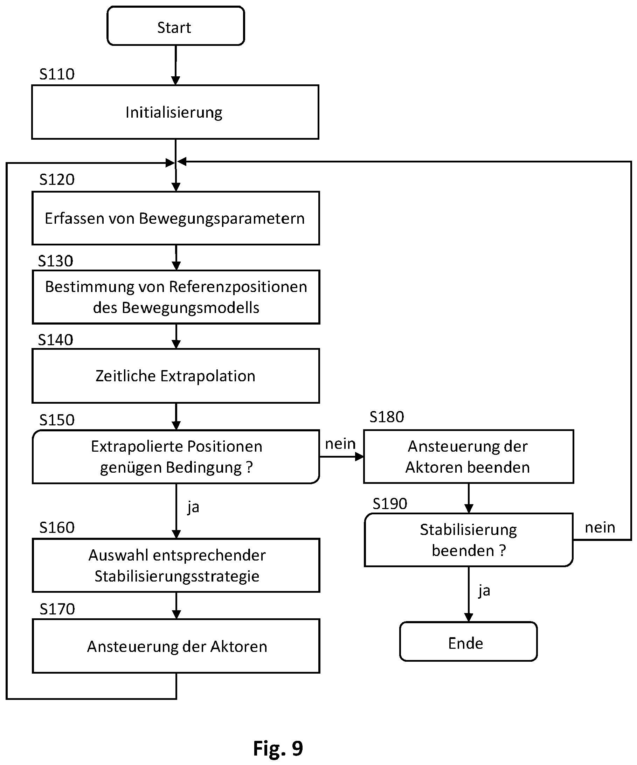

Fig. 9 zeigt schematisch die Schritte eines bewegungsabhängigen Stabilisierungsunterstützungsverfahren zur Aufrechterhaltung der Stabilität eines sich bewegenden Körpers 200 nach einer Ausführungsform.

Nach dem Start wird in Schritt S110 das System 100 initialisiert. Die Initialisierung umfasst beispielsweise das Festlegen von Parametern des Bewegungsmodells, wie beispielsweise von Pendellängen, Massen und Federhärten. Ferner kann die Initialisierung das Festlegen von Sensorpositionen am Körper 200 beinhalten. Die Parameter können dabei auch vorbestimmt sein und auf einer Speichereinheit abgelegt sein.

In Schritt S120 werden die Bewegungsparameter von den Sensoren 110 erfasst und an die Steuereinheit 130 weitergegeben. In Schritt S130 werden anhand der Bewegungsparameter von der Steuereinheit 130 Positionen des biomechanischen Bewegungsmodells bestimmt und in Schritt S140 zeitlich extrapoliert, um eine bevorstehende Instabilität des Körper abschätzen zu können.

Dazu wird in Schritt S150 geprüft, ob die zukünftigen Positionen vorbestimmten Bedingungen, wie sie beispielsweise in Tabelle 1 definiert sind, genügen. Ist eine oder mehrere Bedingungen erfüllt (ja in Schritt S150), wird in Schritt S160 eine entsprechende Stabilisierungsstrategie gewählt und in Schritt S170 durch Ansteuerung entsprechender Aktoren 120 umgesetzt.

Die Erfassung von Bewegungsparametern und das Erkennen einer bevorstehenden Instabilität wird dabei kontinuierlich durchgeführt, so dass nach/während der Ansteuerung der Aktoren weiterhin Bewegungsparameter erfasst und ausgewertet werden.

Genügen die zeitlich extrapolierten Positionen keiner der vorbestimmten Bedingungen (nein in Schritt S150), wird die Ansteuerung der Aktoren in Schritt S180 beendet. Die Beendigung der Ansteuerung wird nur durchgeführt, wenn zum Zeitpunkt der Abschätzung der bevorstehenden Instabilität in Schritt S150 Aktoren 120 angesteuert werden.

In Schritt S190 wird geprüft, ob die Stabilisierungsunterstützung beendet werden soll. Dies kann zum Beispiel durch eine Eingabe durch den Träger des Systems 100 erfolgen. Wenn die Stabilisierungsunterstützung nicht beendet werden soll (nein in Schritt S190), erfolgt eine weitere Erfassung und Auswertung von Bewegungsparametern. Für den Fall, dass die Stabilisierung beendet werden soll (ja in S190), wird das Verfahren zur Stabilisierungsunterstützung entsprechend beendet.

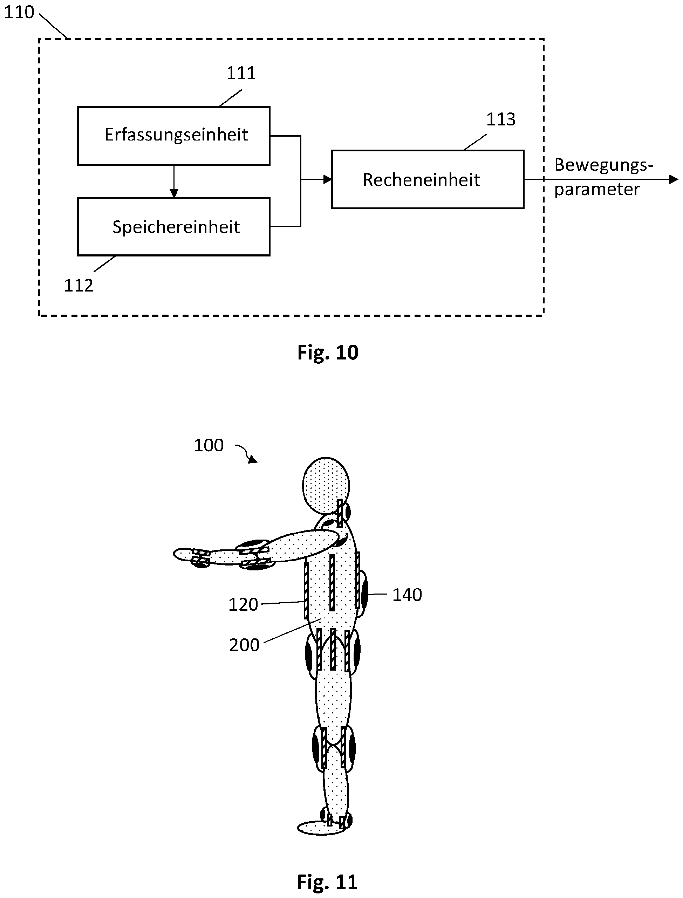

In einer ersten Modifikation der oben beschriebenen Ausführungsform beinhaltet zumindest einer der Sensoren 110 eine Erfassungseinheit 111, eine Speichereinheit 112 und eine Recheneinheit 113, wie in Fig. 10 dargestellt.

Die Erfassungseinheit 111 ist dabei ausgelegt, Sensordaten zu erfassen, die im Folgenden von der Speichereinheit 112 gespeichert werden. Die Recheneinheit 113 verarbeitet vorherige und momentane Sensordaten und gibt ein Ergebnis der Verarbeitung als Bewegungsparameter des Körpers an die Steuereinheit 130 weiter.

Dadurch wird ein Teil der Datenverarbeitung anstatt von der Steuereinheit 130 von den Sensoren 120 selbst durchgeführt. Beispielsweise kann anstelle von Sensordaten wie einer Beschleunigung oder einer Drehrate die Änderung der Beschleunigung oder der Drehrate als Bewegungsparameter erfasst und an die Steuereinheit 130 übertragen werden.

Ferner kann die Speichereinheit 112 auch eine oder mehrere vorbestimmt Bedingungen beinhalten, auf die die Recheneinheit 113 zugreift, um bereits zu ermitteln, ob ein Bewegungsparameter einer der Bedingungen genügt, so dass die Steuereinheit 130 entsprechende Aktoren 120 ansteuern muss, um eine bevorstehende Instabilität zu verhindern. Aus diesem Grund werden solche Sensoren auch als MEM-SSC (Memory-Stabilitätssteuerung und Kontrolle) bezeichnet.

Gemäß einer zweiten Modifikation der beschriebenen Ausführungsform umfasst das System 100 neben der Mehrzahl an Sensoren 110, der Mehrzahl an Aktoren 120 und der Steuereinheit 130 eine Mehrzahl zweiter Aktoren 140, die auch als aktive Aktoren 140 bezeichnet werden.

Fig. 11 zeigt exemplarisch eine solche modifizierte Ausführungsform des Stabilisierungsunterstützungssystems 100, das zusätzlich zweite Aktoren140, die eine aktive Bewegung durchführen, umfasst.

Die aktiven Aktoren 140 sind hier im Bereich des Nackens, des Rückens, der Hüfte, der Knie, der Schultern sowie der Ellenbogen und der Fuß- und Handgelenke der Person 200 angebracht.

In der zweiten Modifikation des Stabilisierungssystems 100 beinhalten die Stabilisierungsstrategien ferner Ansteuervorschriften zur Ansteuerung der zweiten Aktoren 140, zusätzlich zur Ansteuerung der versteifbaren Aktoren 120. Dadurch wird ermöglicht, nicht nur die Bewegungsfreiheit der Person 200 einzuschränken, sondern darüber hinaus aktiv auf die Bewegung der Person 200 Einfluss zu nehmen, und somit einen bereits im Sturz befindlichen Körper wieder zu stabilisieren.

Die zweiten Aktoren können beispielsweise als Hydraulik- oder Pneumatikzylinder, piezoelektrische Antriebe oder klassische elektromagnetische Antriebe realisiert sein.

Gemäß einer weiteren Modifikation werden zur Abschätzung der zukünftigen Stabilität des Körpers nicht nur Bewegungsparameter, die von entsprechenden Sensoren erfasst werden, sondern darüber hinaus weitere Parameter und/oder Messwerte genutzt. Die Gesamtheit der Parameter, die zur Abschätzung der zukünftigen Instabilität des Körpers 200 herangezogen werden, können als einflussnehmende Parameter oder Umgebungsparameter, die auf eine Bewegung Einfluss nehmen, bezeichnet werden.

Umgebungsparameter, die auf eine Bewegung Einfluss nehmen, sind beispielsweise die Bodenbeschaffenheit wie Rutschfestigkeit des Bodens, wie z.B. bei vereisten Böden oder rutschigen Beläge. Ferner kann die Bodenbeschaffenheit berücksichtigt werden. Dabei kann zum Beispiel zwischen eben und uneben, hart, steinig oder weich unterschieden werden.

Die genannte Bodenbeschaffenheit kann als zusätzlicher Parameter in den vorgegebenen Bedingungen definiert sein. Zum Beispiel können die zulässigen Bereiche, Geschwindigkeiten oder Beschleunigungen bei einem rutschigen Boden verringert sein. Des Weiteren kann eine Neigung des Bodens berücksichtigt werden, so dass zum Beispiel zulässige Winkel des Bewegungsmodells zum Lot entsprechend der Bodenneigung verringert oder vergrößert werden.

Zusätzlich kann das System Geräusche berücksichtigen, sowohl bei der Auswahl der Stabilisierungsstrategie als auch bei der Ausprägung derselben. Dazu kann das System 100 zusätzlich mit einem Mikrofon ausgestattet sein, das Geräusche detektiert und an die Steuereinheit 130 weiterleitet. Die Steuereinheit verarbeitet das detektierte Geräuschsignal und wählt eine entsprechende Stabilisierungsstrategie. Beispielsweise kann das Knacken von Gelenken oder die Intensität eines Atemgeräuschs des Trägers des Systems 100 auf dessen momentane körperliche Leistungsfähigkeit hindeuten, die in der Wahl der Stabilisierungsstrategie berücksichtigt wird.

Außerdem kann das System mit weiteren Sensoren ausgestattet sein, mit deren Messwerten auf eine Emotion des Trägers geschlossen werden kann. Zum Beispiel eignen sich hierfür Sensoren zur Messung des Hautwiderstands, des Pulses, des Blutdrucks und/oder von Geräuschen. Die Steuereinheit 130 kann anhand der erfassten Messwerte eine Emotion des Trägers ermitteln, und die zukünftige Stabilitätssituation unter Berücksichtigung der ermittelten Emotion abschätzen. Beispielsweise können die vorbestimmten Bedingungen die ermittelte Emotion als zusätzlichen Parameter enthalten, so dass zum Beispiel bei einer Erregung dem Träger eine erhöhte Bewegungsfreiheit gewährt werden, die zum Beispiel bei ruhiger Emotion zu einer Abschätzung einer Instabilität führen würde.

Insbesondere können die vorbestimmten Bedingungen zur Definition einer bevorstehenden instabilen Situation des Körpers, das biomechanische Bewegungsmodell sowie die Stabilisierungsstrategien unter Berücksichtigung individueller Parameter definiert/initialisiert werden. Dabei können zum Beispiel Parameter wie das Geschlecht, die physische Kondition, das Alter, bekannte körperliche Bewegungseinschränkungen (z.B. durch Schmerzen) oder eine vorliegende Medikation berücksichtigt werden.

Der Körper unterliegt somit beim Stabilisieren der Auswertung von zwei Arten einflussnehmender Parameter. Zum einen basiert die Auswertung auf Daten verschiedener Sensoren und anderen Signalgebern in Echtzeit, zum anderen beruht die Auswertung auf vorhandenen Profildaten.

Alle einflussnehmenden Parameter können im Einzelnen, in Gruppen oder in Summe eine unterschiedliche Rolle bei den vorbestimmten Bedingungen sowie bei entsprechenden Stabilisierungsstrategien spielen.

Das Gesamtkonzept der vorliegenden Erfindung kann somit als adaptiver Sicherheitskäfig beschrieben werden, bei dem durch gezielte Eingriffe durch Ansteuerung der Aktoren 120 ein bevorstehender Sturz des beweglichen Körpers verhindert wird, indem die Bewegungsfreiheit des Körpers in einer Weise eingeschränkt wird, dass eine weitere Bewegung in Richtung des bevorstehenden instabilen Zustands verhindert bzw. gehemmt wird.

Dabei kann die Aktorik zweistufig entworfen sein. So können Gelenke sowie der Rückenbereich durch überlappende Strukturen mit getrennt aktuierbaren Kammern magnetofluidischer Stabilisatoren ausgestattet sein, um eine situationsabhängige passive Stabilisierung durch präzise situative Versteifung durchzuführen. Eine zweite Stufe ermöglicht das Entgegenwirken eines Sturzes sowie ein Aufrichten des Körpers, wenn eine Stabilisierung durch Versteifen nicht ausreichend ist, beispielsweise durch Hydraulik- oder Pneumatikzylinder, piezoelektrischer Antriebe oder klassische elektromagnetische Antriebe.

Zur Energieversorgung des Systems 100 kann beispielsweise ein im System 100 verteiltes Stromversorgungssystem, wie beispielsweise ein Batteriesystem, verwendet werden. Die Verteilung der Energiespeicher erhöht den Komfort und verbessert die passive Stabilität verglichen mit einer zentralen Energieversorgung.

Das System 100 kann ferner eine Programmierschnittstelle (API) umfassen, über die aktuelle Daten zur Beschreibung der drohenden Instabilität übermittelt werden können, so dass diese zur späteren Optimierung der personalisierten Parameter herangezogen werden. Insbesondere kann die Auswirkung einer durchgeführten Stabilisierung ermittelt und gespeichert werden, um dann über die API an eine externe Vorrichtung übermittelt werden kann, die die vorbestimmten Bedingungen sowie die entsprechenden Stabilisierungsstrategien optimiert. Das bewegungsabhängige Stabilisierungsunterstützungssystem 100 kann in ein smartes Textil eingebettet sein.

Zusammenfassend betrifft die vorliegende Erfindung ein bewegungsabhängiges Stabilisierungsunterstützungssystem zur Stabilisierung eines sich bewegenden Körpers, welches eine Mehrzahl an Sensoren und Aktoren sowie eine Steuereinheit umfasst. Die Mehrzahl der Sensoren erfassen kontinuierlich Bewegungsparameter des Körpers, auf Grundlage derer die Steuereinheit ermittelt, ob eine Instabilität des Körpers bevorsteht. Wenn ermittelt wird, dass eine Instabilität bevorsteht, wählt die Steuereinheit eine Stabilisierungsstrategie, nach der die Aktoren angesteuert werden. Bei Ansteuerung versteifen die am Körper angebrachten Aktoren und schränken die Bewegungsfreiheit des Körpers ein, so dass eine Bewegung in Richtung des bevorstehenden instabilen Zustands verhindert bzw. unterdrückt wird. Damit wird der Körper in seiner Stabilisierung unterstützt und ein bevorstehender Sturz verhindert.

## Patentansprüche

1. Bewegungsabhängiges Stabilisierungsunterstützungssystem (100) zur Aufrechterhaltung der Stabilität eines sich bewegenden Körpers (200), umfassend:
eine Mehrzahl an Sensoren (110), die ausgelegt sind, Bewegungsparameter des Körpers (200) zu erfassen;
eine Mehrzahl an Aktoren (120); und
eine Steuereinheit (130), die ausgelegt ist,
anhand der Bewegungsparameter und eines mathematischen Bewegungsmodells zu erkennen, ob eine Instabilität des Körpers (200) bevorsteht, eine Stabilisierungsstrategie aus einer Mehrzahl vorgegebener Stabilisierungsstrategien zu wählen, wenn erkannt wurde, dass eine Instabilität des Körpers (200) bevorsteht,
und die Aktoren (120) entsprechend der gewählten Stabilisierungsstrategie anzusteuern,
wobei
die Aktoren (120) flexibel verformbar sind, und bei Ansteuerung durch die Steuereinheit (130) versteifen wobei
die Steuereinheit (130) so ausgelegt ist, dass sie ferner eine Stützbasis unter Verwendung von Kontaktpunkten der Füße des Körpers (200) und eine jeweilige Standebene des Körpers (200) definiert;
das mathematische Bewegungsmodell eine Position eines Schwerpunkts (CM) des Körpers (200) in Bezug auf die Stützbasis umfasst;
die Steuereinheit (130) ausgelegt ist, Positionen von vorbestimmten Referenzpunkten des mathematischen Bewegungsmodells basierend auf den empfangenen Bewegungsparametern des Körpers zu bestimmen, und
die drohende Instabilität des Körpers basierend auf Trajektorien der Bezugspunkte des mathematischen Bewegungsmodells zu erkennen.

2. Bewegungsabhängiges Stabilisierungsunterstützungssystem nach Anspruch 1, wobei
die Steuereinheit ferner ausgelegt ist, einen Regelungsprozess zur Beendigung der Ansteuerung der Aktoren zur Stabilisierung basierend auf den Bewegungsparametern durchzuführen.

3. Bewegungsabhängiges Stabilisierungsunterstützungssystem nach Anspruch 1 oder 2, wobei
das mathematische Bewegungsmodell auf einer Formalisierung der Bewegung eines einfachen invertierten Pendels oder doppelt invertierter Pendel beruht.

4. Bewegungsabhängiges Stabilisierungsunterstützungssystem nach Anspruch 1 oder 2, wobei
das mathematische Bewegungsmodell auf einer Formalisierung der Bewegung eines einfachen invertierten Pendels auf einer Springfeder oder doppelt invertierter Pendel auf einer Springfeder beruht.

5. Bewegungsabhängiges Stabilisierungsunterstützungssystem nach einem der Ansprüche 1 bis 4, wobei
das mathematische Bewegungsmodell ein erweitertes mathematisches Bewegungsmodell des Körpers ist, das ein menschliches Körpermuskelsystem für Menschen verschiedenen Alters durch ein System invertierter Pendel, verbunden mit elastischen Federn, modelliert.

6. Bewegungsabhängiges Stabilisierungsunterstützungssystem nach Anspruch 1, wobei die
Steuereinheit
die bevorstehende Instabilität des Körpers erkennt, indem anhand von Änderungen zwischen vorherigen und aktuellen Positionen der Referenzpunkte des mathematischen Bewegungsmodells zukünftige Positionen berechnet werden, und
erkennt, dass eine Instabilität des Körpers bevorsteht, wenn die zukünftigen Positionen zumindest einer Bedingung aus einer Mehrzahl vorgegebener Bedingungen genügen.

7. Bewegungsabhängiges Stabilisierungsunterstützungssystem nach einem der Ansprüche 1 bis 6, wobei die Sensoren jeweils eine Erfassungseinheit (111), eine Speichereinheit (112) und eine Recheneinheit (113) umfassen, wobei
die Erfassungseinheit (111) ausgelegt ist, Sensordaten zu erfassen,
die Speichereinheit (112) ausgelegt ist, die Sensordaten zu speichern, und
die Recheneinheit (113) ausgelegt ist, vorherige und momentane Sensordaten zu verarbeiten und ein Ergebnis der Verarbeitung als Bewegungsparameter des Körpers zu erfassen.

8. Bewegungsabhängiges Stabilisierungsunterstützungssystem nach einem der Ansprüche 1 bis 7, wobei
die Steuereinheit nach Erkennen der bevorstehenden Instabilität des Körpers anhand von Bewegungsparametern des Körpers erkennt, ob die Instabilität des Körpers weiterhin vorliegt oder nicht, und
die Aktoren entsprechend ansteuert.

9. Bewegungsabhängiges Stabilisierungsunterstützungssystem nach einem der Ansprüche 1 bis 8, wobei
die Aktoren eine schlauchähnliche Form aufweisen und mit einem magnetisch-sensitiven oder elektrisch-sensitiven Medium gefüllt sind, das bei Ansteuerung durch die Steuereinheit versteift.

10. Bewegungsabhängiges Stabilisierungsunterstützungssystem nach einem der Ansprüche 1 bis 9, wobei
die Aktoren eine magnetorheologische Flüssigkeit beinhalten, die bei Anlegen eines
Magnetfeldes versteift, und
die Aktoren jeweils einen Magnetfeldgenerator umfassen, der bei Ansteuerung durch die Steuereinheit ein Magnetfeld zur Versteifung der magnetorheologischen Flüssigkeit erzeugt.

11. Bewegungsabhängiges Stabilisierungsunterstützungssystem nach einem der Ansprüche 1 bis 10, wobei
die Sensoren inertiale Messeinheiten sind, die Beschleunigungen oder Drehraten als
Bewegungsparameter des Körpers erfassen.

12. Bewegungsabhängiges Stabilisierungsunterstützungssystem nach einem der Ansprüche 1 bis 11, wobei
der sich bewegende Körper eine Person oder ein Roboter menschlicher Gestalt ist.

13. Bewegungsabhängiges Stabilisierungsunterstützungssystem nach Anspruch 12, wobei
die Mehrzahl an Sensoren ausgelegt sind, zumindest an Hüften, Schultern, Händen und
Kopf der Person oder des Roboters menschlicher Gestalt angebracht zu werden

14. Bewegungsabhängiges Stabilisierungsunterstützungssystem nach Anspruch 12 oder 13, wobei die Mehrzahl Aktoren ausgelegt sind, im Bereich von Gelenken und/oder des Torsos der Person oder des Roboters menschlicher Gestalt angebracht zu werden, um die Bewegungsfreiheit der Gelenke und/oder des Torsos durch das Versteifen bei Ansteuerung durch die Steuereinheit einzuschränken.

15. Bewegungsabhängiges Stabilisierungsunterstützungssystem nach einem der Ansprüche 1 bis 14, ferner umfassend
eine Mehrzahl zweiter Aktoren, die ausgelegt sind, bei Ansteuerung durch die
Steuereinheit eine aktive Bewegung durchzuführen, wobei
die Steuereinheit ferner ausgelegt ist, die zweiten Aktoren entsprechend der gewählten
Stabilisierungsstrategie anzusteuern.

16. Bewegungsabhängiges Stabilisierungsunterstützungsverfahren zur Aufrechterhaltung der Stabilität eines sich bewegenden Körpers, umfassend: Erfassung von Bewegungsparametern des Körpers (200),
unter Verwendung einer Vielzahl von Sensoren (110);
Definieren einer Stützbasis durch eine Steuereinheit (130);
Erkennen, ob eine Instabilität des Körpers (200) bevorsteht, anhand der
Bewegungsparameter und eines mathematischen Bewegungsmodells;
Auswählen einer Stabilisierungsstrategie aus einer Mehrzahl vorgegebener Stabilisierungsstrategien, wenn erkannt wurde, dass eine Instabilität des Körpers (200) bevorsteht; und
Versteifen von flexibel verformbaren Aktoren (120) entsprechend der gewählten Stabilisierungsstrategie,
**dadurch gekennzeichnet dass**
die Stützbasis unter Verwendung der Kontaktpunkten der Füße des Körpers (200) und der jeweiligen Standebene des Körpers (200) definiert ist;
das mathematische Bewegungsmodell die Position des Schwerpunkts (CM) des Körpers (200) in Bezug auf die Stützbasis einschließt;
Positionen von festgelegten Referenzpunkten des mathematischen Bewegungsmodells auf der Basis der von Sensoren detektierten Bewegungsparameter des Körpers (200) bestimmt werden, und
die drohende Instabilität des Körpers anhand von Trajektorien der Referenzpunkte des mathematischen Bewegungsmodells in Echtzeit modelliert wird
und diese prognostizierte Instabilität durch Selektion einer geeigneten
Stabilisierungsstrategie verhindert wird.

## Claims

1. Motion-dependent stabilization support system (100) for maintaining the stability of a moving body (200), comprising:
a plurality of sensors (110) designed to detect motion parameters of the body (200);
a plurality of actuators (120); and
a control unit (130) that is designed to detect whether instability of the body (200) is imminent based on the motion parameters and a mathematical motion model,
to select a stabilization strategy from a plurality of predefined stabilization strategies when it is detected that instability of the body (200) is imminent and
to control the actuators (120) according to the selected stabilization strategy,
wherein
the actuators (120) are flexibly deformable and stiffen when actuated by the control unit (130)
wherein the control unit (130) is designed in such a manner that it also defines a support base using contact points of the feet of the body (200) and a respective stance plane of the body (200);
the mathematical motion model comprises a position of a centre of mass (CM) of the body (200) relative to the support base;
the control unit (130) is designed to determine positions of predetermined reference points of the mathematical motion model based on the motion parameters of the body received and
to detect the impending instability of the body based on trajectories of the reference points of the mathematical motion model.

2. Motion-dependent stabilization support system according to Claim 1, wherein
the control unit is further designed to execute a control process for terminating the actuation of the actuators for stabilization based on the motion parameters.

3. Motion-dependent stabilization support system according to Claim 1 or 2, wherein
the mathematical motion model is based on a formalization of the movement of a simple inverted pendulum or double inverted pendulums.

4. Motion-dependent stabilization support system according to Claim 1 or 2, wherein
the mathematical motion model is based on a formalization of the movement of a simple inverted pendulum on a spring or double inverted pendulums on a spring.

5. Motion-dependent stabilization support system according to any one of Claims 1 to 4, wherein
the mathematical motion model is an extended mathematical motion model of the body that models a human body muscle system for people of different ages using a system of inverted pendulums connected to elastic springs.

6. Motion-dependent stabilization support system according to Claim 1, wherein the control unit
detects the impending instability of the body by calculating future positions based on changes between previous and current positions of the reference points in the mathematical motion model and
recognizes that instability of the body is imminent when the future positions meet at least one condition from a plurality of predefined conditions.

7. Motion-dependent stabilization support system according to any one of Claims 1 to 6, wherein the sensors each comprise a detection unit (111), a storage unit (112) and a computing unit (113), wherein
the detection unit (111) is designed to capture sensor data,
the storage unit (112) is designed to store the sensor data and
the computing unit (113) is designed to process previous and current sensor data and capture the result of the processing as motion parameters of the body.

8. Motion-dependent stabilization support system according to any one of Claims 1 to 7, wherein
the control unit, after detecting the impending instability of the body based on motion parameters of the body, determines whether the instability of the body persists or not and
controls the actuators accordingly.

9. Motion-dependent stabilization support system according to any one of Claims 1 to 8, wherein
the actuators have a tube-like shape and are filled with a magnetically-sensitive or electrically-sensitive medium that stiffens when actuated by the control unit.

10. Motion-dependent stabilization support system according to any one of Claims 1 to 9, wherein
the actuators contain a magnetorheological fluid that stiffens when a magnetic field is applied and
the actuators each comprise a magnetic field generator which, when actuated by the control unit, generates a magnetic field to stiffen the magnetorheological fluid.

11. Motion-dependent stabilization support system according to any one of Claims 1 to 10, wherein
the sensors are inertial measurement units that detect accelerations or rotational rates as motion parameters of the body.

12. Motion-dependent stabilization support system according to any one of Claims 1 to 11 wherein
the moving body is a person or a humanoid robot.

13. Motion-dependent stabilization support system according to Claim 12, wherein
the plurality of sensors are designed to be attached at least to the hips, shoulders, hands and head of the person or humanoid robot.

14. Motion-dependent stabilization support system according to Claim 12 or 13, wherein
the plurality of actuators are designed to be attached in the region of the joints and/or torso of the person or humanoid robot, in order to restrict the range of motion of the joints and/or torso by stiffening when actuated by the control unit.

15. Motion-dependent stabilization support system according to any one of Claims 1 to 14, further comprising
a plurality of second actuators which are designed to perform an active movement when actuated by the control unit, wherein
the control unit is further designed to actuate the second actuators according to the selected stabilization strategy.

16. Motion-dependent stabilization support method for maintaining the stability of a moving body, comprising:
detecting motion parameters of the body (200);
using a plurality of sensors (110);
defining a support base through a control unit (130);
detecting whether instability of the body (200) is imminent based on the motion parameters and a mathematical motion model;
selecting a stabilization strategy from a plurality of predefined stabilization strategies when it is detected that instability of the body (200) is imminent; and
stiffening flexibly deformable actuators (120) according to the selected stabilization strategy,
**characterized in that**
the support base is defined using the contact points of the feet of the body (200) and the respective stance plane of the body (200);
the mathematical motion model includes the position of the centre of mass (CM) of the body (200) relative to the support base;
positions of predetermined reference points in the mathematical motion model are determined based on the motion parameters of the body (200) detected by the sensors and
the impending instability of the body is modelled in real-time based on trajectories of the reference points of the mathematical motion model
and this predicted instability is prevented by selecting an appropriate stabilization strategy.

## Revendications

1. Système d'aide à la stabilisation en fonction des mouvements (100) visant à maintenir la stabilité d'un corps en mouvement (200), comprenant :
une pluralité de capteurs (110) conçus pour détecter les paramètres de mouvement du corps (200) ;
une pluralité d'actionneurs (120) ; et
une unité de commande (130) conçue
pour détecter, à l'aide des paramètres de mouvement et d'un modèle mathématique de mouvement, si une
instabilité du corps (200) est imminente,
pour sélectionner une stratégie de stabilisation parmi une pluralité de stratégies de stabilisation prédéterminées lorsqu'il est détecté qu'une instabilité du corps (200) est imminente, et pour commander les actionneurs (120) conformément à la stratégie de stabilisation sélectionnée, dans laquelle
les actionneurs (120) sont déformables de manière flexible et se rigidifient lorsqu'ils sont commandés par l'unité de commande (130), dans lequel
l'unité de commande (130) est conçue pour définir en outre une base de support en utilisant des points de contact des pieds du corps (200) et un plan d'appui respectif du corps (200) ;
le modèle mathématique de mouvement comprend une position d'un centre de gravité (CM) du corps (200) par rapport à la base de support ;
l'unité de commande (130) est conçue pour déterminer la position des points de référence prédéterminés du modèle mathématique de mouvement sur la base des paramètres de mouvement du corps reçus, et
pour détecter l'instabilité imminente du corps sur la base des trajectoires des points de référence du modèle mathématique de mouvement.

2. Système d'aide à la stabilisation en fonction des mouvements selon la revendication 1, dans lequel
l'unité de commande est en outre conçue pour exécuter un processus de régulation visant à déconnecter la commande des actionneurs de stabilisation sur la base des paramètres de mouvement.

3. Système d'aide à la stabilisation en fonction des mouvements selon la revendication 1 ou 2, dans lequel
le modèle mathématique de mouvement est fondé sur une formalisation du mouvement d'un pendule inversé simple ou d'un pendule inversé double.

4. Système d'aide à la stabilisation en fonction des mouvements selon la revendication 1 ou 2, dans lequel
le modèle mathématique de mouvement est fondé sur une formalisation du mouvement d'un pendule inversé simple sur un ressort à boudin ou d'un pendule inversé double sur un ressort à boudin.

5. Système d'aide à la stabilisation en fonction des mouvements selon l'une quelconque des revendications 1 à 4, dans lequel
le modèle mathématique de mouvement est un modèle mathématique de mouvement étendu du corps qui modélise un système musculaire corporel humain pour des personnes d'âges différents grâce à un système de pendules inversés reliés à des ressorts élastiques.

6. Système d'aide à la stabilisation en fonction des mouvements selon la revendication 1, dans lequel l'unité de commande
détecte l'instabilité imminente du corps, en calculant les positions futures à partir des changements entre les positions précédentes et actuelles des points de référence du modèle mathématique de mouvement, et
détecte qu'une instabilité du corps est imminente si les positions futures remplissent au moins une condition parmi une pluralité de conditions prédéterminées.

7. Système d'aide à la stabilisation en fonction des mouvements selon l'une quelconque des revendications 1 à 6, dans lequel les capteurs comprennent chacun une unité de détection (111), une unité de mémoire (112) et une unité de calcul (113), dans lequel
l'unité de détection (111) est conçue pour détecter les données de capteur,
l'unité de mémoire (112) est conçue pour enregistrer les données de capteur, et
l'unité de calcul (113) est conçue pour traiter des données de capteur précédentes et actuelles, et pour enregistrer un résultat du traitement en tant que paramètre de mouvement du corps.

8. Système d'aide à la stabilisation en fonction des mouvements selon l'une quelconque des revendications 1 à 7, dans lequel
l'unité de commande, après avoir détecté l'instabilité imminente du corps, détecte, à l'aide de paramètres de mouvement du corps, si l'instabilité du corps est toujours présente ou non, et
commande les actionneurs en conséquence.

9. Système d'aide à la stabilisation en fonction des mouvements selon l'une quelconque des revendications 1 à 8, dans lequel
les actionneurs présentent une forme tubulaire et sont remplis d'un milieu magnéto-sensible ou électro-sensible qui se rigidifie lorsqu'il est commandé par l'unité de commande.

10. Système d'aide à la stabilisation en fonction des mouvements selon l'une quelconque des revendications 1 à 9, dans lequel
les actionneurs contiennent un liquide magnétorhéologique qui se rigidifie lorsqu'un champ magnétique est appliqué, et
les actionneurs comprennent chacun un générateur de champ magnétique qui, lorsqu'il est commandé par l'unité de commande, génère un champ magnétique permettant de rigidifier le liquide magnétorhéologique.

11. Système d'aide à la stabilisation en fonction des mouvements selon l'une quelconque des revendications 1 à 10, dans lequel
les capteurs sont des unités de mesure inertielles qui détectent des accélérations ou des vitesses de rotation en tant que paramètres de mouvement du corps.

12. Système d'aide à la stabilisation en fonction des mouvements selon l'une quelconque des revendications 1 à 11, dans lequel
le corps en mouvement est une personne ou un robot de forme humaine.

13. Système d'aide à la stabilisation en fonction des mouvements selon la revendication 12, dans lequel
la pluralité de capteurs est conçue pour être placée au moins sur les hanches, les épaules, les mains et la tête de la personne ou du robot de forme humaine.

14. Système d'aide à la stabilisation en fonction des mouvements selon la revendication 12 ou 13, dans lequel
la pluralité d'actionneurs est conçue pour être placée au niveau des articulations et/ou du torse de la personne ou du robot de forme humaine afin de limiter la liberté de mouvement des articulations ou du torse en les rigidifiant lorsqu'ils sont commandés par l'unité de commande.

15. Système d'aide à la stabilisation en fonction des mouvements selon l'une quelconque des revendications 1 à 14, comprenant en outre
une pluralité de deuxièmes actionneurs conçus pour effectuer un mouvement actif lorsqu'ils sont commandés par l'unité de commande, dans lequel
l'unité de commande est en outre conçue pour commander les deuxièmes actionneurs en fonction de la stratégie de stabilisation sélectionnée.

16. Procédé d'aide à la stabilisation en fonction des mouvements visant à maintenir la stabilité d'un corps en mouvement, comprenant :
la détection de paramètres de mouvement du corps (200) ;
en utilisant une pluralité de capteurs (110) ;
la définition d'une base de support par une unité de commande (130) ;
la détection de l'imminence d'une instabilité du corps (200) à partir des paramètres de mouvement et d'un modèle mathématique de mouvement ;
la sélection d'une stratégie de stabilisation à partir d'une pluralité de stratégies de stabilisation prédéterminées s'il est détecté qu'une instabilité du corps (200) est imminente ; et
la rigidification d'actionneurs (120) déformables de manière flexible en fonction de la stratégie de stabilisation sélectionnée,
**caractérisé en ce que**
la base de support est définie en utilisant les points de contact des pieds du corps (200) et le plan d'appui respectif du corps (200) ;
le modèle mathématique de mouvement inclut la position du centre de gravité (CM) du corps (200) par rapport à la base de support ;
la position des points de référence fixes du modèle mathématique de mouvement est déterminée sur la base des paramètres de mouvement du corps (200) détectés par des capteurs, et
l'instabilité imminente du corps est modélisée en temps réel à l'aide des trajectoires des points de référence du modèle mathématique de mouvement
et **en ce que** cette instabilité prévue est évitée en sélectionnant une stratégie de stabilisation appropriée.
